# FASCICULE DE BREVET EUROPEEN

(11) **EP 1 689 695 B1**
(45) Date de publication et mention de la délivrance du brevet: **04.02.2009**
(21) Numéro de dépôt: 04805513.1
(22) Date de dépôt: 23.11.2004
(51) Int. Cl.: C07C 15/08, C07C 5/27, C07C 7/12

(54) **PROCEDE DE PRODUCTION DE PARAXYLENE COMPRENANT UNE ETAPE D'ADSORPTION ET DEUX ETAPES D' ISOMERISATION**
VERFAHREN ZUR HERSTELLUNG VON PARA-XYLOL, UMFASSEND EINEN ABSORPTIONSSCHRITT UND ZWEI ISOMERISIERUNGSSCHRITTE
METHOD FOR PRODUCING PARAXYLENE COMPRISING AN ADSORPTION STEP AND TWO ISOMERIZATION STEPS

(30) Priorité: 26.11.2003 FR 0313964
(43) Date de publication de la demande: 16.08.2006
(73) Titulaire: Institut Français du Pétrole, 92852 Rueil Malmaison Cedex (FR)
(72) Inventeur: LEFLAIVE, Philibert, F-91440 Bures Sur Yvette (FR); WOLFF, Luc, F-69006 Lyon (FR); HOTIER, Gérard, F-92500 Rueil Malmaison (FR)
(86) Numéro de dépôt international: PCT/FR2004/002984
(87) Numéro de publication internationale: WO 2005/054161

(56) Documents cités:
- EP-A- 0 531 191
- FR-A- 2 782 714
- FR-A- 2 792 632

## Description

La production de paraxylène est en constante augmentation depuis trente ans à un rythme annuel moyen de 5 à 6%. Les utilisations du paraxylène sont les productions d'acide téréphtalique et de résines polyéthylène téréphtalate, pour fournir des textiles synthétiques, des cassettes audio et vidéo, des bouteilles et plus généralement des matières plastiques.

Pour satisfaire la demande toujours croissante en paraxylène, les pétrochimistes ont le choix entre pratiquer des augmentations de capacité sur des unités existantes ou construire des unités neuves nécessairement de grande taille puisque le seuil de rentabilité pour de telles unités se situent aux environs de 400 000 t/an.

La présente invention permet de répondre à ces 2 cas de figure et notamment permet de faire face à des augmentations de capacité d'unités existantes (appelés souvent dégoulottate) car les modifications impliquées sont relativement modestes.

En effet tous les équipements de l'unité de production existante peuvent être réutilisés, les ajouts consistant en 1) une vanne tout ou rien par lit ou une vanne rotative modifiée dans la section adsorption de l'unité de séparation en lit mobile simulé, 2) une colonne à distiller le raffinat-2 dans la section distillation de la dite unité, 3) un réacteur d'isomérisation préférentiellement en phase liquide, 4) une unité de recristallisation du paraxylène.

Ces ajouts permettent d'augmenter la production de paraxylène d'environ trente pour cent par rapport à l'unité existante pour un investissement réduit et sans augmentation du coût opératoire unitaire.

### EXAMEN DE L'ART ANTERIEUR:

La production de paraxylène de haute pureté par séparation par adsorption est bien connue de l'art antérieur. L'arrière plan technologique décrivant la production de paraxylène à très haute pureté est illustré dans le brevet EP-A-0 531 191 de la demanderesse.

La production de paraxylène de pureté de l'ordre de 90-95% par adsorption en lit mobile simulé suivi d'une purification finale à plus de 99,7% par cristallisation est également bien connue de l'art antérieur. Elle est illustrée dans les brevets US 5 922 924 et US 5 948 950 de la demanderesse.

Ces brevets enseignent comment concevoir (types de zéolithe, nombre de lits, répartition par zone des lits) et opérer (définition des 3 ratios: solvant sur charge, recyclage sur charge, extrait sur raffinat) l'unité d'adsorption de manière à augmenter la productivité de la zéolithe, baisser le nombre de lit de 24 à un nombre de 8 à 15 suivant les cas, et à réduire le ratio solvant sur charge en assouplissant la contrainte de pureté.

Le brevet français FR 2 792 632 de la demanderesse enseigne qu'il est possible d'utiliser deux unités distinctes d'isomérisation au sein de la boucle traitant les composées aromatiques à 8 atomes de carbone.

Le schéma décrit dans le dit brevet utilise une colonne à distiller, dite colonne de séparation de l'éthylbenzène, qui peut présenter jusqu'à 200 plateaux et doit être opérée avec un taux de reflux très important. En tête de cette colonne à distiller on soutire un premier flux qui est une fraction riche en ethylbenzène que l'on isomérise en phase vapeur dans les conditions suivantes : température supérieure à 300°C, de préférence de 360°C à 480°C, pression inférieure à 2,5 MPa et de préférence de 0,5 à 0,8 MPa (1MPa = 10⁶ Pascal), vitesse spatiale inférieure à 10 h⁻¹, de préférence comprise entre 0.5 et 6 h⁻¹, rapport molaire hydrogène sur hydrocarbure inférieur à 10, et de préférence compris entre 3 et 6.

L'isomérisation en phase liquide est réalisable dans les conditions suivantes : température inférieure à 300°C et de préférence comprise entre 200 et 260°C, pression inférieure à 4 MPa et de préférence comprise entre 2 et 3 MPa, vitesse spatiale inférieure à 10 h⁻¹ et de préférence comprise entre 2 et 4 h⁻¹, le catalyseur étant à base de zéolithe ZSM-5 par exemple.

L'avantage de travailler avec deux isomérisations distinctes est double :
- l'isomérisation en phase liquide à basse température minimise les réactions parasites de craquage, de transalkylation et de dismutation, mais limite la conversion de l'ethylbenzene.
- L'isomérisation en phase vapeur à haute température avec une charge riche en ethylbenzène, favorise la conversion de ce produit, l'équilibre thermodynamique se situant vers 8%.

Mais l'inconvénient du procédé décrit dans le brevet FR 2 792 632 réside dans le fait que la colonne à distiller permettant de séparer les deux flux est à la fois très lourde en investissement et très coûteuse à opérer.

La présente invention permet d'obtenir directement les deux flux alimentant chacun une unité d'isomérisation, par le soutirage au niveau de la colonne d'adsorption de deux raffinats distincts, l'un appelé raffinat intermédiaire qui va alimenter une première isomérisation en phase vapeur après élimination du désorbant, l'autre appelé raffinat-2 qui va alimenter une seconde isomérisation, préférentiellement en phase liquide, après élimination du désorbant.

La présente invention conserve donc les avantages liés à l'utilisation de deux isomérisations distinctes en opérant ces isomérisations sur deux raffinats, ce qui supprime l'inconvénient d'une très lourde colonne à distiller.

Plusieurs documents de l'art antérieur décrivent l'extraction de deux raffinats de composition distincte d'une colonne d'adsorption en lit mobile simulé.

Le brevet US 4 313 015 décrit une telle séparation en lit mobile simulé, sur zéolithe X échangée au baryum, le désorbant étant du diéthylbenzène.

L'extrait obtenu est constitué de paraxylène trop impur (99,44 %) pour être commercialisé aux normes actuelles (standard actuel = 99,7 % mini) et avec un rendement de 97,5 %.

D'autres documents (Brevets FR 2 782 714, FR 2 808 270, FR 2 822 820, FR 2 829 758 et FR 2 833 592 de la demanderesse) illustrent également des séparations utilisant une colonne à lit mobile simulé dont on soutire deux raffinats distincts en plus de l'extrait. Ils visent tous la coproduction de paraxylène de pureté commerciale, de métaxylène et/ou d'orthoxylène.

L'ensemble des documents décrivant le soutirage de deux raffinats distincts d'une colonne d'adsorption en lit mobile simulé, présentent donc des limitations soit en terme de pureté du paraxylène obtenu, soit en terme de rendement de ce paraxylène, lié à la coproduction d'autres isomères.

### DESCRIPTION SOMMAIRE DE L'INVENTION:

Le but principal de l'invention est d'obtenir du paraxylène à une pureté d'au moins 99,7 %. Le procédé selon l'invention est un procédé de production de paraxylène à partir d'une charge contenant des xylènes, de l'éthylbenzène et des hydrocarbures en C9+, comprenant au moins les étapes suivantes:
- une étape d'adsorption dans une unité opérant en lit mobile simulé dont sont issus au moins trois effluents: un extrait constitué essentiellement (c'est-à-dire au moins 50% poids) de paraxylène et de désorbant, une fraction intermédiaire appelée raffinat intermédiaire qui contient essentiellement de l'éthylbenzene, de préférence avec un fort rendement, et une seconde fraction appelée raffinat-2 qui contient essentiellement un mélange de méta et d'orthoxylène, de préférence substantiellement exempt de paraxylène et dont la teneur en éthylbenzène est de préférence inférieure à 5 %,
- une étape d'isomérisation des C8 aromatiques, dans une unité travaillant en phase vapeur et convertissant l'éthylbenzène, pour traiter le raffinat intermédiaire,
- une étape d'isomérisation des C8 aromatiques dans une unité travaillant en phase liquide ou en phase vapeur, de préférence en phase liquide, pour traiter le raffinat-2. En fonction des réglages de l'unité d'adsorption en lit mobile simulé, l'extrait peut être constitué de paraxylène de pureté suffisante pour être commercialisable (i.e. supérieure à 99,7%) et de désorbant, ou bien être constitué de paraxylène à un niveau de pureté insuffisant. Dans ce dernier cas on procède de préférence à la purification par cristallisation de l'extrait préalablement débarrassé du désorbant par distillation.

Cette cristallisation sera préférentiellement conduite entre +10°C et -30°C comme décrit par exemple dans le brevet EP-B1-0 531 191. La liqueur mère issue de la cristallisation peut alors être recyclée au niveau de l'alimentation de l'unité d'adsorption en lit mobile simulé.

Le solvant de lavage des cristaux de paraxylène est par exemple choisi parmi les solvants suivants : n-pentane, eau, paraxylène purifié ou toluène, et la liqueur de lavage résultant de l'étape de lavage peut être recyclée à l'alimentation de la colonne d'adsorption en lit mobile simulé.

Plus particulièrement dans le cas d'un dégoulottage d'une unité existante, l'invention comprend de préférence en combinaison:
- une étape d'adsorption dans une unité opérant en lit mobile simulé ayant la particularité de comporter trois effluents : a) un extrait constitué de désorbant et de paraxylène dont la pureté est de préférence de l'ordre de 90-95% en pourcentages poids, b) un raffinat intermédiaire qui contient l'éthylbenzene avec de préférence un fort rendement, et c) un raffinat-2 qui contient un mélange de méta et d'orthoxylène substantiellement exempt de paraxylène, typiquement moins de 1 %, et dont la teneur en éthylbenzène est de préférence inférieure à 5 %,
- une étape de purification par cristallisation du paraxylène d'au moins une partie de l'extrait,
- une étape d'isomérisation des C8 aromatiques dans une unité travaillant en phase vapeur à haute température, convertissant l'éthylbenzène pour traiter le raffinat intermédiaire,
- une étape d'isomérisation des C8 aromatiques dans une unité travaillant préférentiellement en phase liquide à basse température pour traiter le raffinat-2.

### DESCRIPTION SOMMAIRE DES FIGURES:

La figure 1 représente un schéma du procédé selon l'invention. Les parties en pointillé correspondent à des variantes optionnelles.
La figure 2 représente un schéma de procédé selon l'art antérieur. Les numéros des flux sont les mêmes sur les figures 1 et 2 lorsque ces flux désignent des flux similaires au sens où il s'agit de flux occupant les mêmes positions dans la figure 1 et dans la figure 2.

### DESCRIPTION DETAILLEE DE L'INVENTION:

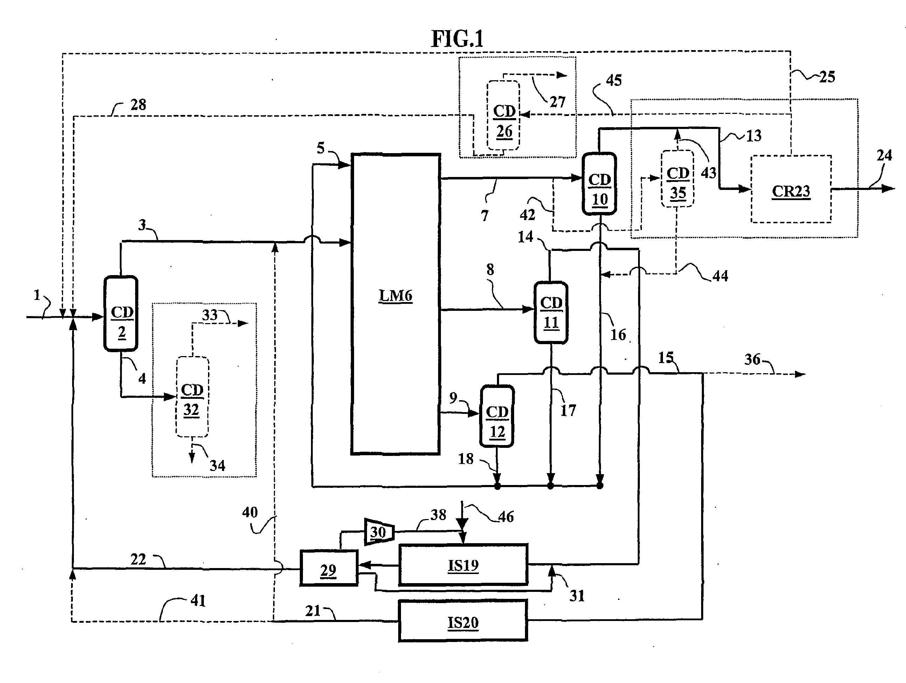

Selon une mise en oeuvre préférée, le procédé de production de paraxylène selon l'invention à partir d'une charge contenant des xylènes, de l'éthylbenzène et des hydrocarbures en C9+, comprend au moins les étapes suivantes (figure 1):
- On envoie la charge (1) dans une colonne de distillation (CD2) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante de l'orthoxylène ;
- On effectue une séparation du mélange de tête (3) en lit mobile simulé dans au moins une colonne de séparation (LM6) contenant une pluralité de lits interconnectés et travaillant en boucle fermée, ladite colonne comprenant au moins cinq zones délimitées par les injections du flux (3) constituant la charge de la colonne (LM6) et du désorbant (5) et les soutirages d'un extrait (7) contenant du paraxylène, d'un raffinat intermédiaire (8) contenant de l'éthylbenzène, et d'un raffinat-2 (9) contenant de l'orthoxylène et du métaxylène, la zone 1 de désorption du paraxylène étant comprise entre l'injection du désorbant (5) et le prélèvement de l'extrait (7), la zone 2 de désorption de l'éthylbenzène, de l'orthoxylène et du métaxylène étant comprise entre le prélèvement de l'extrait (7) et l'injection de la charge d'adsorption (3), la zone 3A d'adsorption du paraxylène étant comprise entre l'injection de la charge (3) et le soutirage du raffinat intermédiaire (8), la zone 3B d'adsorption de l'éthylbenzène étant comprise entre le soutirage de la fraction intermédiaire (8) et le soutirage du raffinat-2 (9), et la zone 5 étant comprise entre le soutirage de raffinat-2 (9) et l'injection du désorbant (5),
- On distille le raffinat intermédiaire (8) dans une colonne CD11 pour éliminer sensiblement tout le désorbant et on soutire une première fraction distillée (14) contenant de l'éthylbenzène
- On distille le raffinat-2 dans une colonne CD12 pour éliminer sensiblement tout le désorbant et on soutire une seconde fraction distillée (15),
- On distille l'extrait (7) dans une colonne CD10, pour récupérer une fraction (13) enrichie en paraxylène,
- On envoie ladite première fraction distillée (14) dans une première zone d'isomérisation des C8 aromatiques (IS19) travaillant en phase vapeur et convertissant l'éthylbenzène pour obtenir un premier isomérat (22)
- On envoie au moins une partie de ladite seconde fraction distillée (15) dans une seconde zone d'isomérisation des xylènes (IS20) pour obtenir un second isomérat (21)
- On envoie le premier isomérat (22) après élimination de ses fractions légères dans un train de séparation (29) vers la colonne de distillation (CD2).
- On recycle le second isomérat (21) soit (flux 40) à la colonne de séparation en lit mobile simulé (LM6), en mélange avec le flux de tête (3) issu de la colonne à distiller (CD2), soit (flux 41) à la colonne à distiller (CD2), en mélange avec la charge (1).

Selon une variante plus préférée de l'invention, la fraction (13) issue de l'extrait (7) est enrichie en paraxylène à au moins 50% de pureté, et est envoyée dans au moins une zone de cristallisation (CR23) pour délivrer des cristaux de paraxylène et une liqueur mère, les cristaux étant séparés de la liqueur mère, éventuellement remis en suspension, lavés et récupérés (flux 24), et la liqueur mère (25) est mélangée à la charge (1) alimentant la colonne de séparation en lit mobile simulé (LM6) via la colonne CD2.

La première zone d'isomérisation (IS19) travaillant en phase gazeuse est généralement opérée dans les conditions suivantes:
- température supérieure à 300°C, de préférence 350°C à 480°C,
- pression inférieure à 4 MPa, de préférence 0,5 à 2 MPa,
- vitesse spatiale horaire (V.V.H.) inférieure à 10 h⁻¹ (10 litres par litre et par heure), de préférence comprise entre 0,5 et 6 h⁻¹,
- catalyseur contenant une zéolithe de type structural EUO, et au moins un métal du groupe VIII,
- rapport molaire H2/hydrocarbures inférieur à 10, de préférence compris entre 3 et 6.

La seconde zone d'isomérisation (IS20) travaille de préférence en phase liquide et est opérée dans les conditions suivantes :
- Température inférieure à 300°C, de préférence 200°C à 260°C,
- Pression inférieure à 4 MPa , de préférence entre 2 et 3 MPa,
- Vitesse spatiale horaire (V.V.H.) inférieure à 10 h⁻¹, de préférence comprise entre 2 et 4 h⁻¹,
- Catalyseur contenant une zéolithe de type ZSM-5.

Il est éventuellement possible d'opérer cette seconde zone d'isomérisation avec un ajout d'hydrogène, de manière par exemple à ce que le rapport molaire H2/hydrocarbures soit inférieur à 10, de préférence compris entre 3 et 6. Un tel fonctionnement avec ajout d'hydrogène est en particulier préféré dans le cas où cette seconde zone d'isomérisation est opérée en phase vapeur.

Selon une autre variante du procédé selon l'invention, le flux (4) de fond de la colonne à distiller (CD2) est distillé dans une colonne de distillation (CD32), pour produire en tête un flux (33) d'orthoxylène de haute pureté, et en fond un flux (34) contenant des hydrocarbures en C9-C10. Selon une sous variante de la variante précédente, le flux contenant l'orthoxylène (33) est recyclé dans la zone d'isomérisation en phase liquide (IS20).

L'adsorbant utilisé dans l'unité de séparation en lit mobile simulé (LM6) est généralement une zéolithe X échangée au baryum ou une zéolithe Y échangée au potassium ou une zéolithe Y échangée au baryum et au potassium.

Le désorbant utilisé dans l'unité de séparation en lit mobile simulé (LM6) est généralement choisi parmi le paradiéthylbenzène, le toluène, le paradifluorobenzène ou des diéthylbenzènes en mélange.Le rapport volumique du désorbant sur la charge dans l'unité de séparation en lit mobile simulé (LM6) est compris entre 0,5 et 2,5, et de préférence compris entre 1,4 et 1,7.

L'unité de séparation en lit mobile simulé (LM6) est opérée à une température comprise entre 20°C et 250°C, de préférence entre 90°C et 210°C, et de manière encore préférée entre 160°C et 200°C, et sous une pression comprise entre la pression de bulle des xylènes à la température opératoire et 2 MPa. La teneur en éthylbenzène de la seconde fraction distillée issue du raffinat-2 (flux 15) atteint au plus 5% poids, et est de préférence inférieure à 1 % poids.

Le catalyseur de l'unité d'isomérisation en phase gazeuse (IS19) peut comprendre une zéolithe de type structural EUO, et au moins un métal du groupe VIII de la classification périodique des éléments dans une proportion pondérale de 0,01% à 2% par rapport au catalyseur.Le catalyseur de l'unité d'isomérisation en phase gazeuse (IS19) peut contenir dans certains cas une zéolithe EU-1 et du platine.

Selon une variante de l'invention, une partie du flux distillé issu du raffinat-2 (flux 15) peut être envoyé dans un ensemble d'unités permettant de produire du métaxylène et /ou de l'orthoxylène à haute pureté.

Le procédé selon l'invention est particulièrement bien adapté à une modification d'une unité existante en vue d'une augmentation de la quantité de paraxylène produite.

Le fonctionnement du procédé selon l'invention, et en particulier la composition des différents flux, est précisé ci-après avec référence à la figure 1. La charge fraîche est introduite par la ligne (1) dans une colonne à distiller (CD2). Cette charge fraîche contient majoritairement des composés C8-aromatiques, xylènes et éthylbenzène, en proportion variable selon l'origine de la coupe. Elle peut contenir éventuellement des impuretés en quantité variable selon l'origine de la charge qui seront essentiellement des composés C9 et C10 aromatiques et des composés paraffiniques et naphtèniques.

La teneur en impuretés naphtèniques ou paraffiniques est avantageusement inférieure à 1 % poids. De manière préférée, cette teneur est inférieure à 0,3 % poids et de manière encore préférée cette teneur est inférieure à 0,1 % poids. La charge peut être issue soit d'une unité d'aromizing, soit d'une unité de dismutation du toluène, soit d'une unité de transalkylation du toluène et des C9 aromatiques.

On ajoute à la charge fraîche un isomérat véhiculé par une ligne (22) et optionnellement une liqueur mère véhiculée par les lignes (25) ou (28) dont la teneur en paraxylène est avantageusement comprise entre 25 et 40%. L'ensemble des flux (1), (22) et (28) ou (25) alimente une colonne à distiller (CD2).

L'effluent de fond (4) de la colonne (CD2) est constitué essentiellement de composés en C9 et C10 aromatiques et éventuellement d'orthoxylène. Optionnellement, le mélange (4) d'orthoxylène et d'hydrocarbures aromatiques en C9-C10 soutiré en fond de la colonne de distillation (CD2), peut être envoyé dans une autre colonne de distillation (CD32) d'où l'on extrait en tête un flux (33) d'orthoxylène de haute pureté (au moins 98,5 %), et en fond un flux (34) contenant des hydrocarbures en C9-C10.

L'effluent de tête (3) de la colonne (CD2) ainsi qu'éventuellement l'isomérat véhiculé par la ligne (21), constituent la charge d'une unité de séparation en lit mobile simulé (LM6). L'unité de séparation en lit mobile simulé (LM6) est alimentée d'une part par la charge véhiculée par la ligne (3), et d'autre part par du désorbant véhiculé par une ligne (5).

Tout type de désorbant peut être utilisé. Le désorbant préféré est le paradiéthylbenzène, cependant d'autres désorbants tels que le toluène, le paradifluorobenzène ou des diéthylbenzènes en mélange peuvent également convenir.

Les effluents de l'unité (LM6) sont un extrait (7), un raffinat intermédiaire (8) et un raffinat-2 (9), ladite unité de séparation comprenant au moins cinq zones délimitées par les injections de charge et de désorbant, et les soutirages de raffinat intermédiaire, de raffinat-2 et d'extrait,
- la zone 1 de désorption du paraxylène, comprise entre l'injection du désorbant (5) et le soutirage de l'extrait (7), comporte de préférence au moins 4 lits d'adsorbant,
- la zone 2 de désorption de l'éthylbenzène, de l'orthoxylène et du métaxylène, comprise entre le soutirage de l'extrait (7) et l'injection de la charge (3), comporte de préférence au moins 9 lits d'adorbants,
- la zone 3A d'adsorption du paraxylène, comprise entre l'injection de la charge (3) et le soutirage du raffinat intermédiaire (8), comporte de préférence au moins 4 lits d'adsorbants,
- la zone 3B d'adsorption de l'éthylbenzène, comprise entre le soutirage du raffinat intermédiaire (8) et le soutirage du raffinat-2 (9), comporte de préférence au moins trois lits d'adsorbants,
- la zone 4 comprise entre le soutirage du raffinat-2 (9) et l'injection du désorbant (5), comporte de préférence au moins deux lits d'adsorbants.

Selon une caractéristique préférée de l'invention, on peut injecter le désorbant dans la zone 1 et la charge dans la zone 3A de la colonne (LM6) dans un rapport volumétrique désorbant sur charge d'au plus 1,7/1, et de manière préférée d'au plus 1,5/1.

L'extrait (7) est constitué essentiellement de toluène, de paraxylène et de désorbant.

Le raffinat intermédiaire (8) est constitué essentiellement de toluène, de métaxylène et d'orthoxylène, d'éthylbenzène, de paraxylène pour la partie non récupéré dans l'extrait, et de désorbant.

Le raffinat-2 (9) est constitué essentiellement de métaxylène et d'orthoxylène et de désorbant. Il est substantiellement exempt de paraxylène et d'éthylbenzène. L'extrait (7) est envoyé dans une colonne à distiller (CD10).

En fond de la colonne (CD10) on soutire par la ligne (16) le désorbant qui est renvoyé dans la zone 1 de la colonne (LM6) par la ligne (5). En tête de la colonne (CD10), on soutire un mélange de paraxylène et de toluène par la ligne (13). Dans le cas où la pureté du paraxylène n'est pas suffisante, le mélange est envoyé vers une unité de cristallisation (CR23).

L'unité (CR23) produit du paraxylène de haute pureté qui est évacué par la ligne (24), et une liqueur mère qui peut être renvoyé soit directement à l'entrée de la colonne à distiller (CD2) par la ligne (25), soit optionnellement, vers une colonne à distiller (CD26) qui permet d'extraire du toluène en tête par la ligne (27) et un mélange de xylènes susceptible de contenir 25 à 40% de paraxylène par la ligne de fond (28) recyclé directement à l'entrée de la colonne à distiller (CD2).

Le raffinat-2 (9) est envoyé dans une colonne à distiller (CD12). En fond de la colonne (CD12) on soutire du désorbant par la ligne (18) qui est renvoyé vers la ligne (5). En tête de la colonne (CD12), on soutire un mélange de métaxylène et d'orthoxylène par une ligne (15), que l'on envoie vers l'isomérisation préférentiellement en phase liquide et à basse température (IS20).

De manière optionnelle, on peut utiliser une partie du flux (15) pour la production de métaxylène ou d'orthoxylène à haute pureté (flux 36), telle que décrite dans la demande de brevet français 01/12777, ou par tout autre moyen connu de l'homme de l'art.

L'unité d'isomérisation (IS20) préférentiellement en phase liquide peut travailler dans les conditions suivantes:
- température inférieure à 300°C, de préférence comprise entre 200 et 260°C,
- pression inférieure à 4 MPa, de préférence comprise entre 2 et 3 MPa,
- vitesse spatiale inférieure à 10 h⁻¹ de préférence comprise entre 2 h⁻¹ et 4 h⁻¹.

Tous les catalyseurs susceptibles d'isomériser les hydrocarbures à 8 atomes de carbone conviennent pour l'unité d'isomérisation (IS20) de la présente invention. De préférence, on utilisera un catalyseur contenant une zéolithe de type ZSM-5.

L'effluent de l'unité d'isomérisation (IS20) est renvoyé par la ligne (21) soit vers la colonne à distiller (CD2), soit directement à l'entrée de l'unité de séparation (LM6) dans le cas où la teneur en composés autres que les C8 -aromatiques est très faible, typiquement de l'ordre de 1 % poids.

Le raffinat intermédiaire est envoyé par une ligne (8) dans une colonne à distiller (CD11). En fond de la colonne (CD11) on renvoie par la ligne (17) le désorbant qui est réintroduit par la ligne (5) dans la zone 1 de l'unité de séparation (LM6). En tête de la colonne (CD11), on soutire par une ligne (14) un mélange de xylènes et d'éthylbenzène. Les effluents de la ligne (14) sont envoyés vers l'unité d'isomérisation (IS19) fonctionnant à haute température en phase vapeur.

L'unité d'isomérisation (IS19) est opérée de préférence dans les conditions suivantes :
- température supérieure à 300°C, de préférence de 360°C à 480°C,
- pression inférieure à 2,5 MPa et de préférence de 0,5 à 0,8 MPa,
- vitesse spatiale inférieure à 10 h⁻¹ de préférence comprise entre 0,5 h⁻¹ et 6 h⁻¹,
- rapport molaire hydrogène sur hydrocarbure inférieur à 10, et de préférence compris entre 3 et 6.

Tous les catalyseurs susceptibles d'isomériser les hydrocarbures à 8 atomes de carbone, zéolithiques ou non, conviennent pour l'unité d'isomérisation (IS19) de la présente invention. De préférence, on utilise un catalyseur contenant une zéolithe acide, par exemple de type structural MFI, MOR, MAZ, FAU et/ou EUO. De manière encore plus préférée, on utilise un catalyseur contenant une zéolithe de type structural EUO et au moins un métal du groupe VIII de la classification périodique des éléments.

De manière préférée, le catalyseur de l'unité d'isomérisation (IS19) renferme de 1 à 70% poids d'une zéolithe de type structural EUO (EU-1 par exemple) comprenant du silicium et au moins un élément T choisi de préférence parmi l'aluminium et le bore dont le rapport Si/T est compris entre 5 et 100. La dite zéolithe est sous forme hydrogène au moins en partie, et la teneur en sodium est telle que le ratio atomique Na/T est inférieur à 0,1. Eventuellement le catalyseur de l'unité d'isomérisation peut contenir entre 0,01 et 2% poids d'étain ou d'indium, et du soufre à raison de 0,5 à 2 atomes par atome de métal du groupe VIII.

L'effluent de l'unité d'isomérisation (IS19) est envoyé dans un train de séparations (29) qui permet de récupérer une partie de l'hydrogène que l'on recycle à l'unité d'isomérisation (IS19) au moyen du compresseur (30) et de la ligne (38). La partie d'hydrogène non recyclé est compensée par un appoint d'hydrogène frais. On récupère une fraction intermédiaire constituée essentiellement d'hydrocarbures paraffiniques et naphténiques que l'on renvoie à l'entrée de l'unité d'isomérisation (IS19) au moyen de la ligne (31). On récupère à l'issue du train de séparation (29) un isomérat constitué des fractions les plus lourdes qui est renvoyé vers la colonne à distiller (CD2) par la ligne (22).

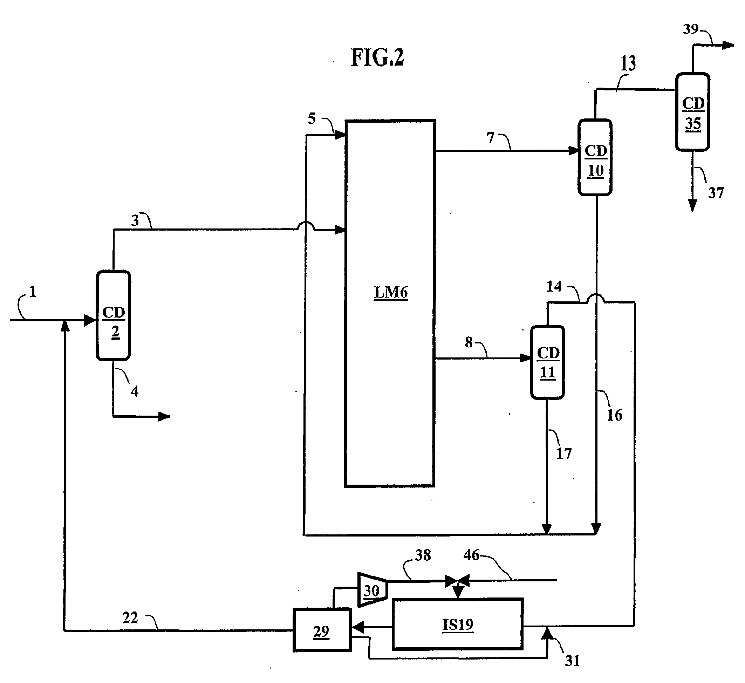

Dans le cas particulier d'un dégoulottage d'une boucle aromatique existante (figure 2), l'invention consiste à augmenter d'environ trente pour cent le débit de charge fraîche et le débit de paraxylène produit tout en continuant à utiliser les équipements principaux de la boucle soit;
1) la colonne de distillation des xylènes (CD2)
2) l'unité de séparation des xylènes (LM6) en lit mobile simulé fonctionnant sur quatre flux principaux (désorbant, charge, extrait contenant le paraxylène haute pureté, un seul raffinat contenant les autres hydrocarbures aromatiques en C8)
3) l'unité d'isomérisation (IS19) alimenté par le raffinat soutiré de la colonne de séparation (LM6) convertissant l'éthylbenzène utilisant par exemple un catalyseur à base de zéolithe de type structural EUO comportant un réacteur, un compresseur de recyclage, une colonne de stabilisation et une colonne permettant de récupérer les naphtènes en C8 et C9 de manière à les recycler à la charge,

Pour réaliser ce dégoulottage selon l'invention, l'unité de séparation en lit mobile simulé (LM6) sera adaptée pour fonctionner avec cinq flux principaux (désorbant, charge, extrait, raffinat intermédiaire et raffinat-2).
L'unité conventionnelle à quatre flux sera modifiée de manière à pouvoir intégrer ce cinquième flux (appelé raffinat-2) et alimentant une unité d'isomérisation spécifique (IS20), l'unique raffinat d'avant le dégoulottage prenant alors l'appellation de raffinat intermédiaire et alimentant toujours l'unité d'isomérisation (IS19). Cette modification sera réalisée soit en ajoutant une vanne tout ou rien par lit, l'ensemble de ces vannes étant relié à un collecteur commun, soit en modifiant la vanne rotative de manière à gérer maintenant cinq flux principaux.

Généralement, la résistance mécanique des plateaux distributeurs séparant les lits est suffisante pour accepter une augmentation de l'ordre de 30% du débit interne. Si tel n'est pas le cas, un déchargement du tamis et un renforcement mécanique des plateaux devra être effectué.

De manière à séparer le raffinat-2 du désorbant, une nouvelle colonne à distiller (CD12) devra également être mise en place. Le raffinat-2 débarrassé du désorbant sera isomérisé dans l'unité d'isomérisation préférentiellement en phase liquide (IS20) telle que décrite plus haut.

Il est à noter que, de préférence, l'isomérat obtenu comme effluent de l'unité (IS20) sera recyclé (flux 40) directement à l'unité d'adsorption (LM6) sans transiter par la colonne de xylènes (CD2) qui généralement ne peut pas accepter une augmentation de la charge de l'ordre de 30%.
Enfin, l'augmentation de l'ordre de 30% du débit de charge et des trafics internes à la colonne d'adsorption (LM6) s'obtient au détriment de la pureté de l'extrait.

Typiquement, la teneur en impuretés dans l'extrait est multipliée par un facteur compris entre 2 et 10 suivant que le ratio désorbant sur charge est ou non maintenu. Il en résulte que seules les unités dont la pureté initiale du paraxylène était proche de 99,9% pourraient continuer à produire du paraxylène commercialisable. Dans le cas le plus général, la pureté du paraxylène tombe en dessous de 99,6% et typiquement en dessous de 99%. Ce dernier doit donc généralement subir une purification finale qui est réalisée dans l'unité de cristallisation (CR23).

D'autre part, la colonne d'extrait (CD10) n'est généralement pas capable d'accepter une augmentation de la charge de l'ordre de 30%. La solution consiste donc à utiliser la colonne de purification du paraxylène (CD35) généralement existante dans la boucle aromatique en parallèle à la colonne d'extrait (CD10).

### EXEMPLES:

L'invention sera mieux comprise à la lecture des deux exemples suivants : L'exemple 1, comparatif, présente une boucle aromatique selon l'art antérieur.
L'exemple 2 présente la même boucle après une augmentation de capacité de 30% réalisée selon l'invention.

### Exemple 1 (comparatif)

Cet exemple illustre l'art antérieur et décrit une boucle aromatique schématisée sur la figure 2 et comportant
- une colonne de xylènes (CD2) permettant d'extraire les aromatiques en C9 et C10 (flux 4) et d'envoyer à l'unité d'adsorption (LM6) un flux (3) constitué essentiellement d'aromatiques en C8.
- une unité d'adsorption en lit mobile simulé (LM6) à 4 zones de laquelle on soutire un extrait (7) et un unique raffinat (8)
- une unité d'isomérisation (IS19) alimentée par une partie (14) du raffinat (8) après élimination du désorbant au moyen de la colonne à distiller (CD11).
- une colonne de purification du paraxylène (CD35) située en aval de la colonne à distillée CD10. En fond de ladite colonne CD35, on soutire du paraxylène (flux 37) à un niveau de pureté supérieur à 99,7 %.

On utilise comme unité de débit le millier de tonnes par an (kt/an).
La charge (1) qui alimente la boucle aromatique est issue du reforming et présente un débit de 460 kt/an. On adjoint à la charge (1) 1540 kt/an d'isomérat (22) recyclés de l'unité d'isomérisation (IS19) convertissant l'éthylbenzène. Le flux résultant est distillé dans la colonne de xylènes (CD2). Les conditions opératoires de cette colonne sont les suivantes :
Colonne : 120 plateaux
Température de tête : 255°C
Pression de tête : 0,95 MPa
Température de fond : 305°C
Pression de fond : 1,05 MPa

On soutire en fond de la colonne (CD2) 20 kt/an d'un mélange de C9 et C10 aromatiques (flux 4) et en tête 1980 kt/an de coupe C8 aromatiques (flux 3) dont la teneur en paraxylène est environ 21%, et la teneur en ethylbenzène environ 10%.

Cette coupe est envoyée dans une unité d'adsorption en lit mobile simulé à quatre zones (LM6) et quatre flux principaux : la charge (flux 3), le désorbant (flux 5), l'extrait (flux 7) et le raffinat (flux 8). Cette unité est composée de 24 lits contenant une zéolithe X échangée au baryum. La température est de 175°C. La configuration est : 5 lits en zone 1, 9 lits en zone 2, 7 lits en zone 3 et 3 lits en zone 4. Le désorbant est constitué à 99% de paradiéthylbenzène, le débit de désorbant (5) est de 3000 kt/an.

Le débit d'extrait (7) en sortie de l'unité d'adsorption (LM6) est de 1200 kt/an, il est envoyé dans une colonne à distiller (CD10) de laquelle on tire en fond 790 kt/an de désorbant (16) recyclé vers l'unité d'adsorption (LM6), et en tête 410 kt/an d'un mélange (13) essentiellement constitué de toluène et de paraxylène.
Ce mélange (13) est envoyé dans une colonne de distillation (CD35) permettant de soutirer 10 kt/an de toluène en tête (flux 39) et 400 kt/an de paraxylène en fond (flux 37).
La pureté du paraxylène obtenu est de 99,8%. Les teneurs en impuretés sont respectivement:
métaxylène: 0,08%
ethylbenzène: 0,08%
orthoxylène: 0,04%.
Le débit de raffinat (8) est de 3780 kt/an. En fond de la colonne (CD11) on soutire 2210 kt/an de désorbant (flux 17) recyclé vers l'unité de séparation (LM6), en tête de colonne on soutire 1570 kt/an de raffinat débarrassé du désorbant (flux 14) dont la teneur en paraxylène est 1 % environ.

On adjoint au flux (14) 160 kt/an d'une coupe paraffinique et naphténique en C8 et C9 (flux 31). Ce mélange est injecté dans le réacteur d'isomérisation (IS19). 130 kt/an d'hydrogène sont recyclés via le compresseur 30 (flux 38).

L'unité d'isomérisation (IS19) travaille aux conditions suivantes:
Température : 385°C
Catalyseur : contient du platine et de la zéolithe EU-1
Vitesse spatiale : 3,5 h⁻¹
Rapport H2/hydrocarbures : 4,4 :1
Pression : 0,9 MPa

La teneur en ethylbenzène du mélange introduit dans l'unité d'isomérisation (IS19) est environ 11,5%. En sortie du réacteur d'isomérisation (IS19) un train de séparation (29) permet de produire 20 kt/an d' un gaz léger contenant essentiellement de l'hydrogène, 20 kt/an de toluène, et 160 kt/an d' une coupe paraffinique et naphténique (flux 31) citée plus haut. La partie la plus lourde de l'isomérat (flux 22), soit 1540 t/an, est recyclée vers l'entrée de la colonne de xylènes (CD2).

### Exemple 2: (selon l'invention)

Cet exemple illustre une application de l'invention consistant à augmenter la capacité de la boucle aromatique décrite dans l'exemple 1 de 30 %. Cet exemple est illustré par la figure 1 selon l'invention.

L'unité de séparation en lit mobile simulé (LM6) est adaptée pour fonctionner avec un nouveau flux appelé raffinat-2, soit cinq flux principaux (le désorbant, la charge, l'extrait, le raffinat intermédiaire et le raffinat-2).

L'unité d'adsorption (LM6) à quatre flux de l'exemple 1 selon l'art antérieur, est donc modifiée de manière à pouvoir intégrer ce cinquième flux, soit en ajoutant une vanne tout ou rien par lit, l'ensemble de ces vannes étant relié à un collecteur commun, soit en modifiant la vanne rotative de manière à gérer maintenant cinq flux principaux.

Généralement, la résistance mécanique des plateaux distributeurs séparant les lits est suffisante pour accepter une augmentation de l'ordre de 30% du débit interne. Si tel n'est pas le cas, un déchargement du tamis et un renforcement mécanique des plateaux doit être effectué. De manière à séparer le raffinat-2 du désorbant, une nouvelle colonne à distiller (CD12) est également mise en place.

On ajoute également à la boucle aromatique de l'exemple 1 une unité de cristallisation (CR23) alimentée par le flux (13) et de laquelle on extrait le paraxylène (flux 24) produit, et une liqueur mère (flux 25) qui est recyclée vers la colonne de xylènes (CD2).

On ajoute encore à la boucle aromatique une unité d'isomérisation travaillant en phase liquide (IS20) à basse température, alimentée par le flux (15) et dont l'effluent (flux 21 et 40) est recyclé vers l'unité d'adsorption (LM6) en mélange avec le flux (3).

La colonne d'adsorption en lit mobile simulé (LM6) est toujours composée de 24 lits contenant une zéolithe X échangée au baryum. Elle comporte à présent 5 zones définies de la manière suivante: 5 lits en zone 1, 9 lits en zone 2, 5 lits en zone 3A, 3 lits en zone 3B et 2 lits en zone 4. La température est de 175°C.

L'unité d'isomérisation en phase liquide (IS20) travaille aux conditions opératoires suivantes:
Pression : 2 MPa
Température : 260°C
Catalyseur : Contient de la zéolithe ZSM-5
Vitesse spatiale : 3 h⁻¹

La charge de la boucle, issue du reforming est de 600 kt/an, soit 30 % de plus que les 460 kt/an de l'exemple 1. On adjoint à la charge (1) 1540 kt/an d'isomérat (flux 22) recyclé de l'unité d'isomérisation (IS19) et 20 kt/an de liqueur mère de cristallisation (flux 28) dont la teneur en paraxylène est de 38%. Le flux résultant est distillé dans la colonne de xylènes (CD2).

On soutire en fond de la colonne (CD2) 30 kt/an de mélange de C9 et C10 aromatiques (flux 4) et en tête 2130 kt/an de coupe C8 aromatiques (flux 3). On adjoint au flux (3) 460 kt/an d'isomérat (flux 40) issu du réacteur d'isomérisation (IS20) et on envoie le flux résultant dans une unité d'adsorption en lit mobile simulé (LM6) à cinq zones et cinq flux principaux. Le désorbant (flux 5) est constitué à 99% de paradiethylbenzène. Le débit de désorbant est de 3900 kt/an.

Le débit d'extrait (flux 7) en sortie de l'unité d'adsorption (LM6) est de 1580 kt/an. Il est envoyé dans les colonnes d'extrait (CD10) et de purification (CD35). Le débit d'extrait envoyé à la colonne (CD10) est de 1200 kt/an. Le débit d'extrait envoyé à la colonne (CD35) est de 380 kt/an.

On soutire en fond de ces colonnes 1040 kt/an de désorbant (flux 16) qui est recyclé vers l'unité d'adsorption en lit mobile simulé (LM6), et en tête 540 kt/an d'un mélange (flux 13) essentiellement constitué de toluène et de paraxylène.

Ce mélange (flux 13) est envoyé dans l'unité de cristallisation (CR23) d'où l'on extrait 20 Kt/an de liqueur mère riche en toluène (flux 25) qui est recyclé à l'entrée de la colonne (CD2), et 520 kt/an de paraxylène de pureté 99,8% (flux 24).
L'unité de cristallisation est constitué de deux zones de cristallisation, la première étant opérée à une température d'environ 10°C, la seconde à une température d'environ -20°C.

Le débit de raffinat intermédiaire (flux 8) est de 3190 kt/an. En fond de la colonne de distillation du raffinat intermédiaire (CD11), on soutire 1600 kt/an de désorbant (flux 17) recyclé vers l'unité de séparation (LM6), et en tête de la colonne (CD11), on soutire 1590 kt/an de raffinat (flux 14), dont la teneur en paraxylène est de 1% environ.

On ajoute au flux (14) 160 kt/an d'une coupe paraffinique et naphténique en C8 et C9 (flux 31). Le mélange des flux (14), (31) est injecté, ainsi qu'un recyclage de 130 kt/an d'hydrogène (38), dans le réacteur d'isomérisation (IS19) qui travaille à haute température en phase vapeur sur un catalyseur contenant du platine et de la zéolithe EU-1.La teneur en ethylbenzène du dit mélange est environ 16,4%.

En sortie du réacteur d'isomérisation (IS19) un train de séparation (29) permet de produire 25 kt/an d'un gaz contenant majoritairement de l'hydrogène, 30 kt/an de toluène et 160 kt/an de la coupe paraffinique et naphténique citée plus haut (flux 31). La partie la plus lourde de l'isomérat (flux 22), soit 1540 t/an, est recyclée vers l'entrée de la colonne de xylènes (CD2). Le débit de raffinat-2 (flux 9) est de 1720 kt/an. En fond de la colonne de distillation du raffinat-2 (CD12), on soutire 1260 kt/an de désorbant (flux 18) recyclé vers l'unité d'adsorption (LM6), et on soutire en tête 460 kt/an un flux (15) dont la teneur en éthylbenzène est de 0,07% et la teneur en paraxylène de 0,15%.

Le mélange (flux 15) est isomérisé à basse température en phase liquide dans une unité d'isomérisation (IS20), et l'isomérat obtenu (flux 21) est renvoyé directement à l'entrée de l'unité d'adsorption (LM6).

L'essentiel des éléments constituants la boucle aromatique initiale est réutilisé, et l'investissement associé à l'accroissement de 30% de production de paraxylène est nettement inférieur à celui qu'aurait nécessité une boucle C8-aromatique neuve de même capacité.

## Revendications

1. Procédé de production de paraxylène à partir d'une charge contenant des xylènes, de l'éthylbenzène et des hydrocarbures en C9+, comprenant au moins les étapes suivantes:
- une étape d'adsorption dans une unité (LM6) opérant en lit mobile simulé dont sont issus au moins trois effluents: un extrait constitué essentiellement de paraxylène et de désorbant, une fraction intermédiaire appelée raffinat intermédiaire qui contient essentiellement de l'éthylbenzene, et une seconde fraction appelée raffinat-2 qui contient essentiellement un mélange de méta et d'orthoxylène,
- une étape d'isomérisation des C8 aromatiques, dans une unité (IS19) travaillant en phase vapeur et convertissant l'éthylbenzène, pour traiter le raffinat intermédiaire,
- une étape d'isomérisation des C8 aromatiques dans une unité (IS20) travaillant en phase liquide ou en phase vapeur pour traiter le raffinat-2.

2. Procédé selon la revendication 1 comprenant en outre, entre l'étape d'adsorption et les étapes d'isomérisation, une étape de purification par cristallisation du paraxylène d'au moins une partie de l'extrait.

3. Procédé selon la revendication 1 ou 2 comprenant au moins les étapes suivantes:
- On envoie la charge (1) dans une colonne de distillation (CD2) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante de l'orthoxylène ;
- On effectue une séparation du mélange de tête (3) en lit mobile simulé dans au moins une colonne de séparation (LM6) contenant une pluralité de lits interconnectés et travaillant en boucle fermée, ladite colonne comprenant au moins cinq zones délimitées par les injections du flux (3) constituant la charge de la colonne (LM6) et du désorbant (5) et les soutirages d'un extrait (7) contenant du paraxylène, d'un raffinat intermédiaire (8) contenant de l'éthylbenzène, et d'un raffinat-2 (9) contenant de l'orthoxylène et du métaxylène, la zone 1 de désorption du paraxylène étant comprise entre l'injection du désorbant (5) et le prélèvement de l'extrait (7), la zone 2 de désorption de l'éthylbenzène, de l'orthoxylène et du métaxylène étant comprise entre le prélèvement de l'extrait(7) et l'injection de la charge d'adsorption (3), la zone 3A d'adsorption du paraxylène étant comprise entre l'injection de la charge (3) et le soutirage du raffinat intermédiaire (8), la zone 3B d'adsorption de l'éthylbenzène étant comprise entre le soutirage de la fraction intermédiaire (8) et le soutirage du raffinat-2 (9), et la zone 5 étant comprise entre le soutirage de raffinat-2 (9) et l'injection du désorbant ( 5),
- On distille le raffinat intermédiaire (8) dans une colonne CD11 pour éliminer sensiblement tout le désorbant et on soutire une première fraction distillée (14) contenant de l'éthylbenzène
- On distille le raffinat-2 dans une colonne CD12 pour éliminer sensiblement tout le désorbant et on soutire une seconde fraction distillée (15),
- On distille l'extrait (7) dans une colonne CD10 pour récupérer une fraction (13) enrichie en paraxylène,
- On envoie ladite première fraction distillée (14) dans une première zone d'isomérisation des C8 aromatiques (IS19) travaillant en phase vapeur et convertissant l'éthylbenzène pour obtenir un premier isomérat (22)
- On envoie au moins une partie de ladite seconde fraction distillée (15) dans une seconde zone d'isomérisation des xylènes (IS20) pour obtenir un second isomérat (21)
- On envoie le premier isomérat (22) après élimination de ses fractions légères dans un train de séparation (29) vers la colonne de distillation (CD2).
- On recycle le second isomérat (21) soit (flux 40) à la colonne de séparation en lit mobile simulé (LM6), en mélange avec le flux de tête (3) issu de la colonne à distiller (CD2), soit (flux 41) à la colonne à distiller (CD2), en mélange avec la charge (1).

4. Procédé selon la revendication 3, dans lequel la fraction (13) issue de l'extrait ( 7) est enrichie en paraxylène à au moins 50% de pureté, et est envoyée dans au moins une zone de cristallisation (CR23) pour délivrer des cristaux de paraxylène et une liqueur mère, les cristaux étant séparés de la liqueur mère, éventuellement remis en suspension, lavés et récupérés (flux 24), et la liqueur mère (25) est mélangée à la charge (1) alimentant la colonne de séparation en lit mobile simulé (LM6) via la colonne à distiller (CD2).

5. Procédé selon l'une des revendications précédentes dans lequel la première zone d'isomérisation (IS19) travaillant en phase gazeuse est opérée dans les conditions suivantes:
- température supérieure à 300°C,
- pression inférieure à 4 MPa,
- vitesse spatiale horaire (V.V.H.) inférieure à 10 h⁻¹,
- catalyseur contenant une zéolithe de type structural EUO, et au moins un métal du groupe VIII,
- rapport molaire H2/hydrocarbures inférieur à 10.

6. Procédé selon l'une des revendications précédentes dans lequel la seconde zone d'isomérisation (IS20) travaille en phase liquide et est opérée dans les conditions suivantes:
- température inférieure à 300°C,
- pression inférieure à 4 MPa ,
- vitesse spatiale horaire (V.V.H.) inférieure à 10 h⁻¹,
- catalyseur contenant une zéolithe de type ZSM-5.

7. Procédé selon l'une des revendications 3 à 6 dans lequel le flux (4) de fond de la colonne à distiller (CD2) est distillé dans une colonne de distillation (CD32), pour produire en tête un flux (33) d'orthoxylène de haute pureté, et en fond un flux (34) contenant des hydrocarbures en C9-C10.

8. Procédé selon la revendication 7 dans lequel le flux contenant l'orthoxylène (33) est recyclé dans la zone d'isomérisation en phase liquide (IS20).

9. Procédé selon l'une des revendications précédentes dans lequel l'adsorbant utilisé dans l'unité de séparation en lit mobile simulé (LM6) est une zéolithe X échangée au baryum ou une zéolithe Y échangée au potassium ou une zéolithe Y échangée au baryum et au potassium.

10. Procédé selon l'une des revendications précédentes, dans lequel le désorbant utilisé dans l'unité de séparation en lit mobile simulé (LM6) est choisi parmi le paradiéthylbenzène, le toluène, le paradifluorobenzène ou des diéthylbenzènes en mélange.

11. Procédé selon l'une des revendications précédentes, dans lequel le rapport volumique du désorbant sur la charge dans l'unité de séparation en lit mobile simulé (LM6) est compris entre 0,5 et 2,5.

12. Procédé selon l'une des revendications précédentes, dans lequel l'unité de séparation en lit mobile simulé (LM6) est opérée à une température comprise entre 20°C et 250°C, et sous une pression comprise entre la pression de bulle des xylènes à la température opératoire et 2 MPa.

13. Procédé selon l'une des revendications 3 à 12 dans lequel la teneur en éthylbenzène de la seconde fraction distillée issue du raffinat-2 (flux 15) atteint au plus 5% poids.

14. Procédé selon l'une des revendications précédentes, dans lequel le catalyseur de l'unité d'isomérisation en phase gazeuse (IS19) comprend une zéolithe de type structural EUO et au moins un métal du groupe VIII de la classification périodique des éléments dans une proportion pondérale de 0,01 % à 2% par rapport au catalyseur.

15. Procédé selon l'une des revendications précédentes dans lequel le catalyseur de l'unité d'isomérisation en phase gazeuse (IS19) contient une zéolithe EU-1 et du platine.

16. Procédé selon l'une des revendications 3 à 15 dans lequel une partie du flux distillé issu du raffinat-2 (flux 15) est envoyé dans un ensemble d'unités permettant de produire du métaxylène et /ou de l'orthoxylène à haute pureté.

## Claims

1. A process for producing para-xylene from a feed containing xylenes, ethylbenzene and C9+ hydrocarbons, comprising at least the following steps:
• a step for adsorption in a unit (LM6) operating as a simulated moving bed from which at least three effluents are produced: an extract essentially constituted by para-xylene and desorbant; an intermediate fraction, termed the intermediate raffinate, which essentially contains ethylbenzene, and a second fraction, termed the 2-raffinate, which essentially contains a mixture of meta- and ortho-xylene;
• a step for isomerization of C8 aromatics, in a unit (IS 19) operating in the vapour phase and converting ethylbenzene, to treat the intermediate raffinate;
• a step for isomerization of C8 aromatics in a unit (IS20) operating in the liquid phase or in the vapour phase, to treat the 2-raffinate.

2. A process according to claim 1, further comprising, between the adsorption step and the isomerization steps, a step for purifying the para-xylene of at least a portion of the extract by crystallization.

3. A process according to claim 1 or claim 2, comprising at least the following steps:
• sending the feed (1) to a distillation column (CD2) from which a mixture (3) is extracted overhead comprising the major portion of the meta-xylene, para-xylene, ethylbenzene and at least a portion of the ortho-xylene, and from which a stream (4) of C9-C10 hydrocarbons and the remaining portion of the ortho-xylene are extracted from the bottom;
• separating the overhead mixture (3) in a simulated moving bed in at least one separation column (LM6) containing a plurality of interconnected beds and operating as a closed loop, said column comprising at least five zones defined by the injections of the stream (3) constituting the feed for the column (LM6) and the desorbant (5) and the withdrawals of an extract (7) containing para-xylene, an intermediate raffinate (8) containing ethylbenzene and a 2-raffinate (9) containing ortho-xylene and meta-xylene, the para-xylene desorption zone 1 being included between the desorbant injection (5) and the extract removal (7); the ethylbenzene, ortho-xylene and meta-xylene desorption zone 2 being included between the extract removal zone (7) and the adsorption feed injection (3); the para-xylene adsorption zone 3A being included between the feed injection (3) and the intermediate raffinate withdrawal (8); the ethylbenzene adsorption zone 3B being included between the intermediate fraction withdrawal (8) and the 2-raffinate withdrawal (9); and the zone 5 being included between the 2-raffinate withdrawal (9) and the desorbant injection (5);
• distilling the intermediate raffinate (8) in a column (CD11) to eliminate substantially all of the desorbant and withdrawing a first distilled fraction (14) containing ethylbenzene;
• distilling the 2-raffinate in a column (CD12) to eliminate substantially all of the desorbant and withdrawing a second distilled fraction (15);
• distilling the extract (7) in a column (CD10) to recover a fraction (13) which is enriched in para-xylene;
• sending said first distilled fraction (14) to a first zone for isomerizing C8 aromatics (IS 19) operating in the vapour phase and converting ethylbenzene to obtain a first isomerate (22);
• sending at least a portion of said second distilled fraction (15) to a second xylene isomerization zone (IS20) to obtain a second isomerate (21);
• sending the first isomerate (22), after eliminating its light fractions, into a separation train (29) towards the distillation column (CD2);
• recycling the second isomerate (21) either (stream 40) to the simulated moving bed separation column (LM6) as a mixture with the overhead stream (3) from the distillation column (CD2) or (stream 41) to the distillation column (CD2) as a mixture with the feed (1).

4. A process according to claim 3, in which the fraction (13) from the extract (7) is enriched in para-xylene with a purity of at least 50%, and is sent to at least one crystallization zone (CR23) to deliver para-xylene crystals and a mother liquor, the crystals being separated from the mother liquor, optionally taken up in suspension, washed and recovered (stream 24) and the mother liquor (25) is mixed with the feed (1) supplying the simulated moving bed separation column (LM6) via the column (CD2).

5. A process according to one of the preceding claims, in which the first isomerization zone (IS 19) operating in the gas phase is operated under the following conditions:
• a temperature of more than 300°C;
• a pressure of less than 4 MPa;
• an hourly space velocity (HSV) of less than 10 h⁻¹;
• a catalyst containing a zeolite with structure type EUO and at least one group VIII metal;
• a H₂/hydrocarbon molar ratio of less than 10.

6. A process according to one of the preceding claims, in which the second isomerization zone (IS20) operating in the liquid phase is operated under the following conditions:
• a temperature of less than 300°C;
• a pressure of less than 4 MPa;
• an hourly space velocity (HSV) of less than 10 h⁻¹;
• a catalyst containing a ZSM-5 type zeolite.

7. A process according to one of claims 3 to 6, in which the stream (4) from the bottom of the distillation column (CD2) is distilled in a distillation column (CD32) to produce an overhead stream (33) of high purity ortho-xylene, and a bottom stream (34) containing C9-C10 hydrocarbons.

8. A process according to claim 7, in which the stream containing ortho-xylene (33) is recycled to the isomerization zone in the liquid phase (IS20).

9. A process according to one of the preceding claims, in which the adsorbent used in the simulated moving bed separation unit (LM6) is a barium-enriched X zeolite or a potassium-enriched Y zeolite or a barium- and potassium-enriched Y zeolite.

10. A process according to one of the preceding claims, in which the desorbant used in the simulated moving bed separation unit (LM6) is selected from para-diethylbenzene, toluene, para-difluorobenzene or a mixture of diethylbenzenes.

11. A process according to one of the preceding claims, in which the volume ratio of the desorbant to the feed in the simulated moving bed separation unit (LM6) is in the range 0.5 to 2.5.

12. A process according to one of the preceding claims, in which the simulated moving bed separation unit (LM6) is operated at a temperature in the range 20°C to 250°C and at a pressure in the range from the bubble pressure of xylenes at the operating temperature to 2 MPa.

13. A process according to one of claims 3 to 12, in which the ethylbenzene content of the second distilled fraction from the 2-raffinate (stream 15) is at most 5% by weight.

14. A process according to one of the preceding claims, in which the gas phase isomerization unit (IS 19) comprises a zeolite with structure type EUO and at least one metal from group VIII of the periodic table in a proportion of 0.01% to 2% by weight with respect to the catalyst.

15. A process according to one of the preceding claims, in which the catalyst from the gas phase isomerization unit (IS19) contains an EU-1 zeolite and platinum.

16. A process according to one of claims 3 to 15, in which a portion of the distilled stream from the 2-raffinate (stream 15) is sent to a set of units which can produce high purity meta-xylene and/or ortho-xylene.

## Patentansprüche

1. Verfahren zur Herstellung von Paraxylol aus einer Beschickung, die Xylole, Ethylbenzol und Kohlenwasserstoffe mit 9+ C-Atomen umfasst, das mindestens die folgenden Schritte umfasst:
- einen Adsorptionsschritt in einer Einheit (LM6), die mit simuliertem Bewegtbett betrieben wird, aus dem mindestens drei Abflüsse hervorgehen: ein Extrakt, der im Wesentlichen aus Paraxylol und Desorbens besteht, eine Intermediärfraktion, die Intermediärraffinat genannt wird, die hauptsächlich Ethylbenzol enthält, und eine zweite Fraktion, die Raffinat-2 genannt wird, die hauptsächlich ein Gemisch aus Meta- und Orthoxylol enthält,
- einen Isomerisierungsschritt der C8-Aromaten, in einer Einheit (IS19), die in der Dampfphase arbeitet und das Ethylbenzol umwandelt, um das Intermediärraffinat zu behandeln,
- einen Isomerisierungsschritt der C8-Aromaten, in einer Einheit (IS20), der in der Flüssigphase oder in der Dampfphase arbeitet, um das Raffinat-2 zu behandeln,

2. Verfahren nach Anspruch 1, das ferner zwischen dem Adsorptionsschritt und den Isomerisierungsschritten einen Reinigungsschritt durch Kristallisierung des Paraxylols mindestens eines Teils des Extrakts umfasst.

3. Verfahren nach Anspruch 1 oder 2, das mindestens die folgenden Schritte umfasst:
- die Beschickung (1) wird in eine Destillationssäule (CD2) geschickt, aus der am Kopf ein Gemisch (3) abgezogen wird, das den Großteil des Metaxylols, Paraxylol, Ethylbenzol und mindestens einen Teil des Orthoxylols umfasst, und aus der am Boden ein Strom (4) aus Kohlenwasserstoffen mit 9-10 C-Atomen und der verbleibende Teil des Orthoxylols abgezogen wird;
- eine Trennung des Kopfgemischs (3) im simulierten Bewegtbett in mindestens einer Trennsäule (LM6) durchgeführt wird, die eine Vielzahl von miteinander verbundenen Betten enthält und im geschlossenen Kreislauf arbeitet, wobei die Säule mindestens fünf Zonen umfasst, die durch die Injektionen des Stroms (3) begrenzt sind, der aus der Beschickung der Säule (LM6) und dem Desorbens (5) besteht, und den Entnahmen eines Extrakts (7), der Paraxylol enthält, eines Intermediärraffinats (8), das Ethylbenzol enthält, und eines Raffinats-2 (9), das Orthoxylol und Metaxylol enthält, wobei die Zone 1 der Desorption des Paraxylols im Bereich zwischen der Injektion des Desorbens (5) und der Ableitung des Extrakts (7) liegt, die Zone 2 der Desorption des Ethylbenzols, des Orthoxylols und des Metaxylols im Bereich zwischen der Entnahme des Extrakts (7) und der Injektion der Adsorptionsbeschickung (3) liegt, die Zone 3A der Adsorption des Paraxylols im Bereich zwischen der Injektion der Beschickung (3) und der Entnahme des Intermediärraffinats (8) liegt, die Zone 3B der Adsorption des Ethylbenzols im Bereich zwischen der Entnahme der Intermediärfraktion (8) und der Entnahme des Raffinats-2 (9) liegt, und die Zone 5 im Bereich zwischen der Entnahme des Raffinats-2 (9) und der Injektion des Desorbens (5) liegt,
- das Intermediärraffinat (8) in einer Säule CD11 destilliert wird, um im Wesentlichen das gesamte Desorbens zu entfernen und eine erste destillierte Fraktion (14) abgezogen wird, die Ethylbenzol enthält
- das Raffinat-2 in einer Säule CD12 destilliert wird, um im Wesentlichen das gesamte Desorbens zu entfernen und eine zweite destillierte Fraktion (15) abgezogen wird,
- der Extrakt (7) in einer Säule CD10 destilliert wird, um eine Fraktion (13) zu gewinnen, die mit Paraxylol angereichert ist,
- die erste destillierte Fraktion (14) in eine erste Zone zur Isomerisierung der C8-Aromaten (IS19) geschickt wird, die in Dampfphase arbeitet und das Ethylbenzol umwandelt, um ein erstes Isomerat (22) zu erhalten
- mindestens ein Teil der zweiten destillierten Fraktion (15) in eine zweite Zone zur Isomerisierung der Xylole (IS20) geschickt wird, um ein zweites Isomerat (21) zu erhalten,
- das erste Isomerat (22) nach der Entfernung seiner leichten Fraktionen in einen Trennzug (29) zur Destillationssäule (CD2) geschickt wird.
- das zweite Isomerat (21) entweder (Strom 40) als Gemisch mit dem Kopfstrom (3), der aus der Destillationssäule (CD2) hervorgeht, zur Trennsäule im simulierten Bewegtbett (LM6), oder (Strom 41) als Gemisch mit der Beschickung (1) zur Destillationssäule (CD2) recycelt wird.

4. Verfahren nach Anspruch 3, wobei die Fraktion (13), die aus dem Extrakt (7) hervorgeht, mit Paraxylol mit mindestens 50 % Reinheit angereichert ist, und in mindestens eine Kristallisierungszone (CR23) geschickt wird, um Paraxylolkristalle und eine Mutterlauge zu ergeben, wobei die Kristalle von der Mutterlauge getrennt, gegebenenfalls resuspendiert, gewaschen und gewonnen werden (Strom 24), und die Mutterlauge (25) mit der Beschickung (1) gemischt wird, die der Trennsäule im simulierten Bewegtbett (LM6) über die Destillationssäule (CD2) zugeführt wird.

5. Verfahren nach einem der vorhergehenden Ansprüche, wobei die erste Zone zur Isomerisierung (IS19), die in Gasphase arbeitet, unter den folgenden Bedingungen betrieben wird:
- Temperatur größer als 300 °C,
- Druck kleiner als 4 MPa,
- Raumgeschwindigkeit pro Stunde (H.S.V.) kleiner als 10 h⁻¹,
- Katalysator, der einen Zeolithen des Strukturtyps EUO und mindestens ein Metall der Gruppe VIII enthält,
- Molverhältnis H₂/Kohlenwasserstoffe kleiner als 10.

6. Verfahren nach einem der vorhergehenden Ansprüche, wobei die zweite Zone zur Isomerisierung (IS20) in Flüssigphase arbeitet und unter den folgenden Bedingungen betrieben wird:
- Temperatur kleiner als 300 °C,
- Druck kleiner als 4 MPa,
- Raumgeschwindigkeit pro Stunde (H.S.V.) kleiner als 10 h⁻¹,
- Katalysator, der einen Zeolithen des Typs ZSM-5 enthält.

7. Verfahren nach einem der Ansprüche 3 bis 6, wobei der Bodenstrom (4) der Destillationssäule (CD2) in einer Destillationssäule (CD32) destilliert wird, um am Kopf einen Strom (33) aus hochreinem Orthoxylol und am Boden einen Strom (34) herzustellen, der Kohlenwasserstoffe mit 9-10 C-Atomen enthält.

8. Verfahren nach Anspruch 7, wobei der Strom, der das Orthoxylol (33) enthält, in die Zone zur Isomerisierung in der Flüssigphase (IS20) recycelt wird.

9. Verfahren nach einem der vorhergehenden Ansprüche, wobei das Adsorptionsmittel, das in der Einheit zur Trennung im simulierten Bewegtbett (LM6) verwendet wird, ein X-Zeolith ist, der mit Barium ausgetauscht ist, oder ein Y-Zeolith, der mit Kalium ausgetauscht ist, oder ein Y-Zeolith, der mit Barium und Kalium ausgetauscht ist.

10. Verfahren nach einem der vorhergehenden Ansprüche, wobei das Desorbens, das in der Einheit zur Trennung im simulierten Bewegtbett (LM6) verwendet wird, ausgewählt ist aus Paradiethylbenzol, Toluol, Paradifluorbenzol oder Diethylbenzolgemischen.

11. Verfahren nach einem der vorhergehenden Ansprüche, wobei das Volumenverhältnis des Desorbens zur Beschickung in der Einheit zur Trennung im simulierten Bewegtbett (LM6) im Bereich zwischen 0,5 und 2,5 liegt.

12. Verfahren nach einem der vorhergehenden Ansprüche, wobei die Einheit zur Trennung im simulierten Bewegtbett (LM6) bei einer Temperatur im Bereich zwischen 20 °C und 250 °C und unter einem Druck im Bereich zwischen dem Blasendruck der Xylole bei Betriebstemperatur und 2 MPa betrieben wird.

13. Verfahren nach einem der Ansprüche 3 bis 12, wobei der Gehalt an Ethylbenzol der zweiten destillierten Fraktion, die aus dem Raffinat-2 (Strom 15) hervorgeht, höchstens 5 Gew.-% erreicht.

14. Verfahren nach einem der vorhergehenden Ansprüche, wobei der Katalysator der Einheit zur Isomerisierung in der Gasphase (IS19) einen Zeolithen des Strukturtyps EUO und mindestens ein Metall der Gruppe VIII des Periodensystems der Elemente in einem Gewichtsanteil von 0,01 % bis 2 %, bezogen auf den Katalysator, umfasst.

15. Verfahren nach einem der vorhergehenden Ansprüche, wobei der Katalysator der Einheit zur Isomerisierung in der Gasphase (IS19) einen Zeolithen EU-1 und Platin enthält.

16. Verfahren nach einem der Ansprüche 3 bis 15, wobei ein Teil des destillierten Stroms, der aus dem Raffinat-2 hervorgeht (Strom 15) in eine Gruppe von Einheiten geschickt wird, die es ermöglicht, Metaxylol und/oder Orthoxylol mit hoher Reinheit herzustellen.
